# EUROPEAN PATENT APPLICATION

(11) **EP 3 904 882 A1**
(43) Date of publication of application: **03.11.2021**
(21) Application number: 19902534.7
(22) Date of filing: 26.12.2019
(51) Int. Cl.: G01N 33/68, G01N 33/551, G01N 27/30, G01N 27/407, G01N 33/12

(54) **GRAPHENE CHANNEL MEMBER COMPRISING CADAVERINE OLFACTORY RECEPTOR AND SENSOR COMPRISING SAME**

(30) Priority: 26.12.2018 KR 20180169873
(71) Applicant: Korea Research Institute of Bioscience and Biotechnology, Daejeon 34141 (KR); Korea Institute of Science and Technology, Seoul 02792 (KR); Seoul National University R & DB Foundation, Seoul 08826 (KR)
(72) Inventor: KWON, Oh Seok, Daejeon 34141 (KR); SONG, Hyun Seok, Seoul 02792 (KR); PARK, Tai Hyun, Seoul 06668 (KR); KIM, Kyung Ho, Daejeon 34141 (KR); KIM, Jin Yeong, Daejeon 34141 (KR); MOON, Dong Seok, Seoul 08104 (KR); PARK, Seon Joo, Daejeon 34141 (KR); PARK, Chul Soon, Daejeon 34141 (KR)
(74) Representative: Regimbeau
(86) International application number: PCT/KR2019/018526
(87) International publication number: WO 2020/138982

(57) **Abstract**

The present invention relates to a graphene channel member comprising a graphene film and a cadaverine olfactory receptor immobilized to the graphene film, a graphene transistor comprising: a substrate; the graphene channel member; and a pair of electrodes, and a bio sensor comprising same.

## Description

### TECHNICAL FIELD

The present invention relates to a graphene channel member comprising a cadaverine olfactory receptor and a sensor comprising same.

### BACKGROUND ART

The conventional discrimination platform of meat freshness is a method of monitoring the change of electric resistance by physisorption based on a metal oxide thin film or a nano material, which has a suitable structure for detecting small molecules (hydrogen, ethanol, ammonia, methanol, toluene, or the like), but there are current limits as follows.

1. Selectivity - Since the change of electric resistance by physisorption is monitored, at the same concentration, both material A and material B may react, but at different concentrations (in case of excessive amount of material B), though material A has relatively higher sensitivity, the reactivity of material B is monitored to increase.

2. Indirect discrimination of meat freshness - If meat decays in practice, a gas generated includes cadaverine and putrescine, but these are supramolecular materials, and there is absolutely no reaction by the conventional physisorption-based platform. Accordingly, the conventional platform had a limitation in that it had to detect ammonia, toluene, or the like as an indirect measure.

The conventional precedent research for detecting cadaverine has been reported carbon nanotubes combined with cadaverine receptors by the present team of researchers for the first time (Park, Taehyeon, et al., ACS Nano, 2017, 11, 11847-11855). The detection limit by the carbon nanotubes is 10 pM, but an amount of the carbon nanotubes mounted on an electrode is not constant, and insufficient reproducibility of experimental results became a stumbling block in the way of commercialization. Accordingly, in order to overcome such problems, a next-generation nano material capable of replacing the carbon nanotube is required as it is.

### DISCLOSURE OF THE INVENTION

### TECHNICAL PROBLEM

The present invention is made to overcome the above-described limits of the conventional detector for meat freshness, and provides a sensor having reproducibility and high sensitivity by using a graphene channel member including a graphene film and a cadaverine olfactory receptor (Taar13c) immobilized to the graphene film.

### TECHNICAL SOLUTION

The present invention provides a graphene channel member comprising a graphene film and a cadaverine olfactory receptor immobilized to the graphene film.

In addition, the present invention provides a graphene transistor comprising: a substrate; the graphene channel member; and a pair of electrodes.

In addition, the present invention provides a bio sensor comprising the graphene transistor.

### ADVANTAGEOUS EFFECTS

In the case where the graphene film and the cadaverine olfactory receptor (trace amine-associated receptor 13c (Taar13c)) immobilized to the graphene film of the present invention is used as a graphene transistor, the detection limit of cadaverine produced during the decay of meat may be improved, and effects of detecting with high sensitivity and reproducibility may be achieved.

Particularly, compared to the conventional sensor using a cadaverine receptor in combination with carbon nanotube, effects of achieving excellent detection efficiency, detection limit and reproducibility and detecting gas phase cadaverine as well as liquid phase cadaverine simultaneously may be achieved.

In addition, the graphene transistor including the graphene channel member of the present invention may be manufactured in a USIM chip type and applied in a miniaturized bio sensor (portable electronic gas sensor, or the like), and effects of very simply and accurately discriminating the freshness of meat may be achieved.

### BRIEF DESCRIPTION ON DRAWINGS

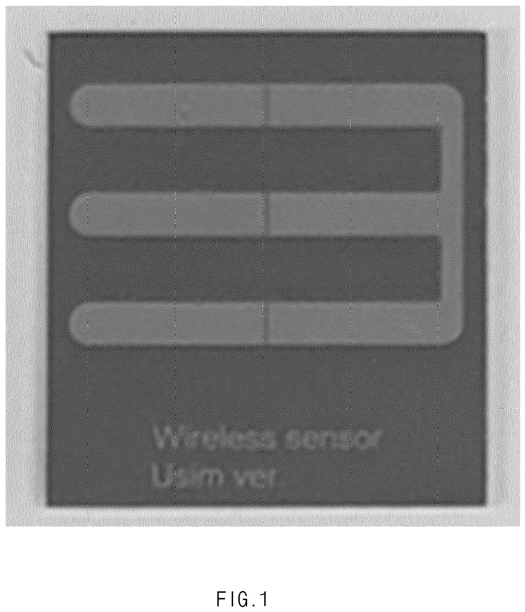

FIG. 1 shows a graphene transistor according to an embodiment of the present invention.

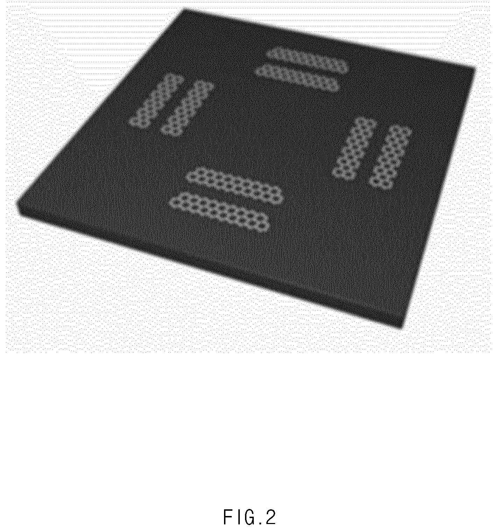

FIG. 2 shows minutely patterned graphene film according to an embodiment of the present invention.

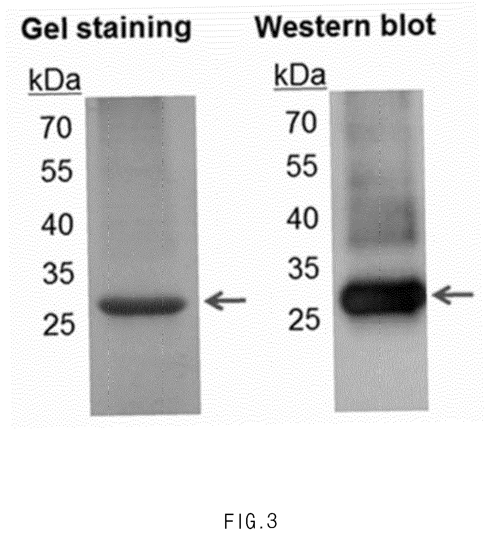

FIG. 3 shows analyzed results of the expressed and purified cadaverine olfactory receptor according to Preparation Example 1 of the present invention by gel staining and western blot.
FIG. 4 shows measured results of the detection limit of cadaverine of the graphene transistor of Example 2 according to Experimental Example 1 of the present invention.
FIG. 5 shows photographic images observing the decay degrees of beef with the naked eye according to Experimental Example 2 of the present invention.
FIG. 6 shows detection results of cadaverine produced from beef using the graphene transistor of Example 2 according to Experimental Example 2 of the present invention.
FIG. 7 shows evaluation results of cadaverine selectivity of the graphene transistor of Example 2 according to Experimental Example 3 of the present invention.
FIG. 8 shows results shown in NO₂, VBN and VOC detectors which may be commercialized in a bio sensor according to an embodiment of the present invention.
FIG. 9 is a diagram showing a bio sensor including a graphene transistor according to an embodiment of the present invention.
FIG. 10 shows ¹H-NMR spectrum of Carbene Compound 1 according to Preparation Example 2.
FIG. 11 shows ¹H-NMR spectrum of Carbene Compound 2 according to Preparation Example 3.
FIG. 12 shows ¹H-NMR spectrum of Carbene Compound 3 according to Preparation Example 4.
FIG. 13 shows ¹H-NMR spectrum of Carbene Compound 4 according to Preparation Example 5.
FIG. 14 shows ¹H-NMR spectrum of Carbene Compound 5 according to Preparation Example 6.
FIG. 15 shows measured results of cadaverine detection limit using the graphene transistor of Comparative Example 1 according to Comparative Experimental Example 1.

### BEST MODE FOR CARRYING OUT THE INVENTION

Hereinafter, the present invention will be described in detail.

### 1. Graphene channel member

The present invention provides a graphene channel member including a graphene film and a cadaverine olfactory receptor immobilized to the graphene film.

The cadaverine olfactory receptor may react with liquid phase cadaverine and/or gas phase cadaverine.

The cadaverine olfactory receptor may include a trace amine-associated receptor 13c (Taar13c). The Taar13c is one of a receptor related to a trace amount of amine of zebrafish and reacts very sensitively to cadaverine.

The cadaverine olfactory receptor may have an immobilized type to the surface of the graphene film by a physical bond, or may have an immobilized type by a chemical bond.

Particularly, the immobilization of the cadaverine olfactory receptor to the surface of the graphene film by a physical bond may be immobilization through physisorption. In this case, the cadaverine olfactory receptor may be immobilized to the surface of the graphene film through physisorption without a separate linker. For example, after treating to the surface of the graphene film, the cadaverine olfactory receptor may be immobilized by a physisorption method of reacting at temperature conditions of about 1 to 10°C for 12 to 48 hours.

Alternatively, the immobilization of the cadaverine olfactory receptor to the surface of the graphene film by a chemical bond may be bonding (immobilization) by including a carbene compound represented by Formula 1 or Formula 2 below as a linker. That is, the carbene compound may play the role of a medium for chemically immobilizing the cadaverine olfactory to the graphene film. In case of using such a carbene compound as a linker, and the cadaverine olfactory receptor is immobilized to the graphene film, there are advantages in achieving excellent stability to the change of external environment.

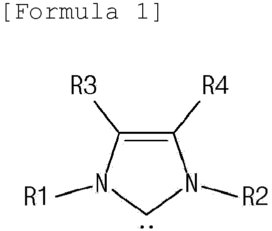

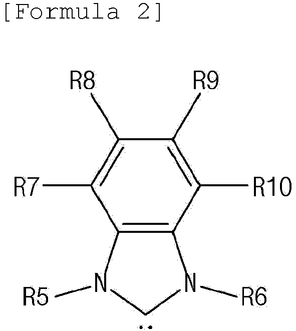

In Formulae 1 and 2,
R1, R2, R5 and R6 are the same or different, and are each independently hydrogen, an alkyl group of 1 to 20 carbon atoms, a cycloalkyl group of 3 to 20 carbon atoms, an aryl group of 6 to 30 carbon atoms, or a heteroaryl group of 2 to 30 carbon atoms,
R3, R4, R7, R8, R9 and R10 are the same or different, and are each independently hydrogen, an alkyl group of 1 to 20 carbon atoms, a cycloalkyl group of 3 to 20 carbon atoms, an aryl group of 6 to 30 carbon atoms, a heteroaryl group of 2 to 30 carbon atoms, or a structure represented by Formula 3 below, or two or more adjacent substituents among R7 to R10 are combined to form a hydrocarbon ring,
at least one of R3 and R4 is a structure represented by Formula 3 below, and
in the case where at least one of R7 to R10 has a structure represented by Formula 3 below, or two or more adjacent substituents among R7 to R10 are combined to form a hydrocarbon ring, at least one hydrogen which is bonded to carbon forming the hydrocarbon ring is substituted with a structure represented by Formula 3 below,

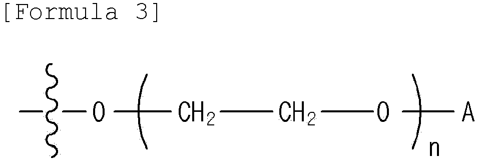

in Formula 3,
n is a repeating number of the unit in a parenthesis and an integer of 1 to 30, and
A is an alkyl group of 1 to 20 carbon atoms, including a nitrogen (N) atom, or a heteroaryl group of 2 to 30 carbon atoms, including a nitrogen (N) atom.
R1, R2, R5 and R6 may be the same or different, and may be each independently hydrogen, an alkyl group of 1 to 20 carbon atoms or an aryl group of 6 to 30 carbon atoms.
R1, R2, R5 and R6 may be the same or different, and may be each independently hydrogen, an alkyl group of 1 to 10 carbon atoms or an aryl group of 6 to 10 carbon atoms.
R1, R2, R5 and R6 may be the same or different, and may be each independently hydrogen, or an alkyl group of 1 to 10 carbon atoms.
R1, R2, R5 and R6 may be the same or different, and may be each independently hydrogen, isopropyl or benzyl.

At least one of R1 and R2 and at least one of R5 and R6 may be the same or different, and may be each independently an alkyl group of 1 to 20 carbon atoms or an aryl group of 6 to 30 carbon atoms.

At least one of R1 and R2 and at least one of R5 and R6 may be the same or different, and may be each independently isopropyl or benzyl.

R3, R4, R7, R8, R9 and R10 may be the same or different, and may be each independently hydrogen, an alkyl group of 1 to 20 carbon atoms, a cycloalkyl group of 3 to 20 carbon atoms, an aryl group of 6 to 30 carbon atoms, a heteroaryl group of 2 to 30 carbon atoms or a structure represented by Formula 3, or two or more adjacent substituents among R7 to R10 may be combined to form a hydrocarbon ring.

R3, R4, R7, R8, R9 and R10 may be the same or different, and may be each independently hydrogen, or a structure represented by Formula 3, or two or more adjacent substituents among R7 to R10 may be combined to form a hydrocarbon ring.

In the case where two or more adjacent substituents among R7 to R10 are combined to form a hydrocarbon ring, at least one hydrogen which is bonded to carbon forming the hydrocarbon ring may be substituted with a structure represented by Formula 3.

At least one of R3 and R4 may have the structure represented by Formula 3. In addition, at least one of R7 to R10 may have the structure represented by Formula 3.

The n is a repeating number in the parenthesis and may be an integer of 1 to 30, preferably, 1 to 10. More preferably, n may be 1 to 3.

The A may be an alkyl group of 1 to 20 carbon atoms, including a nitrogen (N) atom, or a heteroaryl group of 2 to 30 carbon atoms, including a nitrogen (N) atom. Particularly, the A may be azide, phthalimide, or amine.

In the present invention, an "adjacent" group may mean a substituent substituted at an atom which is directly connected with an atom at which a corresponding substituent is substituted, a substituent sterically at the nearest position to a corresponding substituent, or another substituent substituted at an atom at which a corresponding substituent is substituted. For example, two substituents substituted at ortho positions in a benzene ring, and two substituents substituted at the same carbon in an aliphatic ring may be interpreted as "adjacent" groups to each other.

The alkyl group may be a linear chain or a branched chain, and may have 1 to 20 carbon atoms, preferably, 1 to 10 carbon atoms. More preferably, the carbon number may be 1 to 6. Particular examples of the alkyl group may include a methyl group, an ethyl group, a propyl group, an n-propyl group, an isopropyl group, a butyl group, an n-butyl group, an isobutyl group, a tert-butyl group, a sec-butyl group, an 1-methylbutyl group, an 1-ethylbutyl group, a pentyl group, an n-pentyl group, an isopentyl group, a neopentyl group, a tert-pentyl group, a hexyl group, an n-hexyl group, an 1-methylpentyl group, a 2-methylpentyl group, a 4-methyl-2-pentyl group, a 3,3-dimethyl butyl group, a 2-ethylbutyl group, a heptyl group, an n-heptyl group, an 1-methylhexyl group, a cyclopentylmethyl group, a cyclohexylmethyl group, an octyl group, an n-octyl group, a tert-octyl group, an 1-methylheptyl group, a 2-ethylhexyl group, a 2-propylpentyl group, an n-nonyl group, a 2,2-dimethylheptyl group, an 1-ethylpropyl group, an 1,1-dimethylpropyl group, an isohexyl group, a 4-methylhexyl group, a 5-methylhexyl group, or a benzyl group, without limitation.

The cycloalkyl group may have 3 to 20 carbon atoms, preferably, 3 to 10 carbon atoms. Particular examples of the cycloalkyl group may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group or a cyclooctyl group, without limitation.

The aryl group may have 6 to 30 carbon atoms, preferably, 6 to 10 carbon atoms. The aryl group may be a monocyclic aryl group or a polycyclic aryl group. Particular examples of the monocyclic aryl group may include a phenyl group, a biphenyl group or a terphenyl group, and particular examples of the polycyclic aryl group may include a naphthyl group, an anthracenyl group, a phenanthryl group, a pyrenyl group, a perylenyl group, a chrysenyl group, a fluorenyl group, or a triphenylene group, without limitation.

The heteroaryl group may be an aromatic ring group including one or more selected from N, O, P, S, Si and Se as a heteroatom and having 2 to 30 carbon atoms, preferably, 2 to 20 carbon atoms. Particular examples of the heteroaryl group may include a thiophene group, a furan group, a pyrrole group, an imidazole group, a thiazole group, an oxazole group, an oxadiazole group, a triazole group, a pyridyl group, a pyrimidyl group, a triazine group, a triazole group, an acridyl group, a quinolinyl group, a quinazoline group, a quinoxalinyl group, a phthalazinyl group, an isoquinoline group, an indole group, a carbazole group, a benzoxazole group, a benzimidazole group, a benzothiazole group, a benzocarbazole group, a benzothiophene group, a dibenzothiophene group, or a benzofuranyl group, without limitation.

In addition, the alkyl group, cycloalkyl group, aryl group, heteroaryl group or hydrocarbon ring may be further substituted or unsubstituted with an alkyl group, cycloalkyl group, aryl group or heteroaryl group.

After treating the carbene compound to the graphene film, a temperature of 100°C or higher may be applied so that unshared electron pair of the carbene compound may make a chemical bond (covalent bond) with the graphene film.

The cadaverine olfactory receptor may be chemically bonded to the carbene compound, for example, a functional group at the terminal group of the carbene compound and the functional group of the cadaverine olfactory receptor may make chemical bond by a coupling agent (for example, 4-(4,6-dimethoxy-1,3,5-triazin-2-yl)-4-methylmorpholinium tetrafluoroborate (DMTMM), N-(3-dimethylaminopropyl)-N'-ethylcarbodiimide hydrochloride and N-hydroxysuccinimide (EDC-NHS), glutaraldehyde, or the like).

Particularly, a sensor using after combining the conventional cadaverine receptor and carbon nanotube, had a detection limit of 10 pM, and the amount of carbon nanotubes mounted on an electrode was not constant, and accordingly, there were problems in reproducibility of the experimental results of a graphene transistor. However, in the graphene channel member of the present invention, a cadaverine olfactory receptor is physically or chemically bonded to a graphene film having high charge mobility, and improved detection efficiency by about 1,000 times or more, particularly, 10,000 times or more, more particularly, 100,000 times or more than a carbon nanotube-based sensor, may be shown, and there are advantages in having a detection limit of 0.1 fM which is further sensitive sensor performance than a case of using the conventional carbon nanotube, and detecting gas phase cadaverine as well as liquid phase cadaverine simultaneously.

The graphene film may have a single layer or a bi-layer. If a graphene film with a bi-layer is used, the sensitivity of a graphene transistor may be degraded due to the reduction of surface resistance, and in this regard, a graphene film with a single layer is preferably included.

The graphene film may be patterned, particularly, minutely patterned. On the surface of the patterned graphene film, the cadaverine olfactory receptor may be bonded (immobilized). For example, the graphene film may be patterned into diverse shapes such as a circle, triangle, square, pentagon, and hexagon (honeycomb). In case of patterning the graphene film as described above, patterns of the graphene film with various shapes may be provided, a bio sensor may be miniaturized and easy to carry, and requirement on the design with various shapes of a graphene transistor may be met.

On the surface of the patterned graphene film, the cadaverine olfactory receptor may be bonded. In this case, the cadaverine olfactory receptor may be immobilized to the surface of the graphene film by a physical bond through physisorption, or may be immobilized to the graphene film by a chemical bond through the carbene compound represented by Formula 1 or Formula 2 as a linker.

As described above, the graphene channel member includes the graphene film, and in case of using graphene as such a channel member, high current flows in an off state when a voltage is not applied to a gate, the on/off ratio of an operation current is very low, and there are advantages in manufacturing a transistor having high performance.

In this case, the thickness of the graphene film may be 0.1 to 1 nm, and this may mean the thickness of a graphene film having a single layer. If the thickness of the graphene film satisfies the above-described range, the properties of high conductivity and high charge mobility may be shown, and a graphene transistor with high sensitivity may be manufactured.

### 2. Graphene transistor

In addition, the present invention provides a graphene transistor including the graphene channel member.

The graphene transistor of the present invention includes: a substrate; the above-described graphene channel member; and a pair of electrodes.

The substrate plays the role of a support for supporting the constituents of the graphene transistor of the present invention and may use an insulating inorganic substrate including a Si substrate, a glass substrate, a GaN substrate, a silica (SiO₂) substrate, or the like, a metal substrate including Ni, Cu, W, or the like, or a plastic substrate. In case of using the insulating substrate, the silica (SiO₂) substrate, or a silicon wafer is preferable in regard of excellent affinity with the graphene channel member.

In addition, the substrate may be selected from diverse materials on which the deposition of graphene is possible, for example, may be composed of a material including silicon-germanium, silicon carbide (SiC), or the like, and may include an epitaxial layer, a silicon-on-insulator layer, a semiconductor-on-insulator layer, or the like.

The graphene channel member may be formed on the substrate.

Particularly, a graphene film may be formed by growing graphene on the substrate by a chemical vapor deposition method using a hydrocarbon gas as a carbon source.

The graphene film may be formed by using, for example, a chemical vapor deposition method, and by using this method, graphene having a single layer to several layers having excellent crystallinity may be obtained into a large area. The chemical vapor deposition method is a method for growing graphene by separating carbon atoms by adsorbing, decomposing or reacting a carbon precursor in a gas or vapor state, having high kinetic energy onto the surface of a substrate, and making the carbon atoms form atomic bonds from each other.

The chemical vapor deposition method may be at least one selected from the group consisting of plasma enhanced chemical vapor deposition (PECVD), atmospheric pressure chemical vapor deposition (APCVD) and low pressure chemical vapor deposition (LPCVD), and in view of depositing on a large area while minimizing defects, a preferable chemical vapor deposition method is LPCVD.

By a particular method of the chemical vapor deposition, for example, a metal catalyst such as nickel, copper, aluminum and iron is deposited using a sputtering apparatus and an electron beam evaporation apparatus on a wafer having a silicon oxide layer to form a metal catalyst layer, this is put in a reactor together with a gas including carbon such as CH₄ and C₂H₂ and heated so that carbon is absorbed into the metal catalyst layer, and then, cooled to separate carbon from the metal catalyst layer for crystallization, and finally, the metal catalyst layer is removed to form a graphene film.

However, the method of forming the graphene film is not limited to the chemical vapor deposition method, and the graphene film may be formed using various methods.

For example, a graphene film may be formed by a physical stripping method for forming graphene by stripping one layer from a graphite crystal composed of many layers by a mechanical force, a chemical stripping method utilizing oxidation-reduction properties, or an epitaxial synthetic method by heating a material in which carbon is adsorbed or included such as SiC at a high temperature state of 1,500°C.

The one pair of electrodes may be a source electrode and a drain electrode separately formed on the graphene film to apply a voltage to the graphene channel member.

The source electrode and drain electrode may be electrically connected through the graphene film, may include a material having conductivity, and may be formed using, for example, a metal, a metal alloy, a conductive metal oxide, a conductive metal nitride, or the like.

The source electrode and the drain electrode may each independently include at least one selected from the group consisting of Cu, Co, Bi, Be, Ag, Al, Au, Hf, Cr, In, Mn, Mo, Mg, Ni, Nb, Pb, Pd, Pt, Re, Rh, Sb, Ta, Te, Ti, W, V, Zr, Zn, and combinations thereof, without limitation. Considering contact with graphene and easiness of etching, Au, or a Cr/Au alloy are preferable.

The one pair of electrodes may be formed by a method well-known in the art and may be formed by a deposition method including, for example, photolithography, thermal deposition, E-beam deposition, plasma enhanced chemical vapor deposition (PECVD), low pressure chemical vapor deposition (LPCVD), physical vapor deposition (PVD), sputtering, atomic layer deposition (ALD), or the like.

In the graphene channel member, a cadaverine olfactory receptor may be directly bonded (immobilized) to the graphene film by physisorption, or a cadaverine olfactory receptor may be bonded (immobilized) via the carbene compound. With respect to the graphene channel member, the cadaverine olfactory receptor, the carbene compound and the graphene film, the same explanation described above may be applied.

In the case where the cadaverine olfactory receptor is bonded (immobilized) to the graphene film by including the carbene compound as a linker, the carbene compound bonded to the graphene film may form a linker layer as a single layer type, and the cadaverine olfactory receptor immobilized to the carbene compound may also form a receptor layer as a single layer type.

If the linker layer is formed into a single layer, excellent charge mobility, transparency and/or flexibility inherent in graphene may be obtained, and effects of blocking noise signals due to the approach of external nonspecific charges may be achieved.

In addition, the linker layer may have a thickness of 0.1 to 2 nm. If the thickness of the linker layer is smaller than 0.1 nm, there are problems of increasing resistance, and if the thickness is greater than 2 nm, there are problems of degrading transparency.

### 3. Bio sensor

Another aspect of the present invention provides a bio sensor including the above-described graphene transistor.

The bio sensor according to the present invention uses semiconductor characteristics by which current flowing through the graphene film between the source and drain electrodes changes by electric field effects.

Particularly, if a cadaverine olfactory receptor formed on the surface of the graphene film reacts with cadaverine, surrounding electric field changes, and due to this, a current value flowing through the graphene film between the source electrode and the drain electrode changes concurrently, and by a method of measuring such current change, a target material may be detected.

Such a bio sensor has excellent sensitivity, specificity, promptness and/or portability by using such a graphene transistor, and particularly, due to the high charge carrier mobility and conductivity properties of graphene by using the graphene film as a channel layer, excellent sensitivity and real-time sensing performance are shown. Accordingly, the detection limit of cadaverine generated during the decay of meat may be improved, and effects of high sensitivity and reproducibility may be achieved.

In addition, in the case where the above-described linker layer is formed on the graphene film in the graphene transistor, and a cadaverine olfactory receptor layer bonded thereto is present in the channel area of the graphene transistor, the sensitivity of the sensor may be even further improved, and doping treatment and attaching of the cadaverine olfactory receptor may be performed simultaneously, thereby simplifying a process.

Further, the above-described graphene transistor may be formed into a USIM chip type and may be applied in a miniaturized bio sensor (portable electronic gas sensor, or the like), and the freshness of meat may be simply and accurately discriminated in real-time, and this bio sensor may be utilized in various food industries and environment evaluation industries.

Hereinafter, the present invention will be explained in more detail referring to preferred embodiments.

However, these embodiments are only for explaining the present invention more particularly, and the scope of the present invention is not limited thereto.

### <Preparation Example 1>

### 1. Gene cloning of ApoA-I and Taar13c

In order to express ApoA-I and Taar13c proteins from *Escherichia coli (E. coli),* ApoA-I and Taar13c genes were cloned first.

Particularly, the ApoA-I gene was designed so as to include 6xHis and stop codon gene, and was amplified by PCR (primer sequence: 5' CAC CAG GAG ATA TAC ATA TGA AAG CTG CGG TGC TGA CC 3', 5' CTA GTG GTG GTG GTG GTG GTG CTG GGT GTT GAG CTT CTT AGT GTA 3') using human genomic DNA.

The Taar13c gene was amplified through PCR (primer sequence: 5'-CAC CAG GAG ATA TAC ATA TGA TGC CCT TTT GCC ACA AT 3', 5' TGA ACT CAA TTC CAA AAA TAA TTT ACA C-3') using zebrafish cDNA. Amplified PCR products were inserted into pET-DEST42 vector (Invitrogen, USA) using a gateway cloning system (Invitrogen, USA).

The Taar13c gene was cloned into a pcDNA3 mammalian expression vector using the amplified PCR product (primer; 5' ATG AAT TCA TGG ATT TAT CAT CAC AAG AAT 3', 5' ATC TCG AGT CAA ACC GTA AAT AAA TTG ATA 3').

### 2. Expression of ApoA-I in E. coli and purification

BL21(DE3) *E. coli* cells bearing a pET-DEST42/ApoA-I construct were cultured in 1 L of Luria-Bertani (LB) medium (+50 µg/mL ampicillin) at 37°C, and then grown until the OD₆₀₀ value of the cells reached 0.5. In addition, isopropyl thiogalactoside (IPTG) was added to a final concentration of 1 nM to induce the overexpression of ApoA-I.

After 3 hours, the cells were centrifuged (7000 g, 20 min, 4°C), resuspended in a lysis buffer (20 mM Tris-HCl, 0.5 M NaCl, 20 mM imidazole, pH 8.0), and then disrupted by sonication (5 s on/off, 5 min).

The disrupted cell lysate was centrifuged at 12,000 g for 30 minutes at 4°C, and ApoA-I in a supernatant was collected and loaded on HisTrap HP column (GE Healthcare, Sweden) through FPLC (GE Healthcare).

Then, the column was washed with a washing buffer (20 mM Tris-HCl, 50 mM imidazole, 0.5 M NaCl, pH 8.0), and ApoA-I was separated using a separation buffer (20 mM Tris-HCl, 400 mM imidazole, 0.5 M NaCl, pH 8.0) and dialyzed against a HEPES buffer I (20 mM HEPES-NaOH, 100 mM NaCl, 20 mM cholate, 1 mM EDTA, pH 8.0) using a HiTrap HP desalting column (GE Healthcare, Sweden). The protein thus dialyzed was stored at 4°C until used.

### 3. Expression and purification of Taar13c

BL21 (DE3) cells transformed with pET-DEST42/Taar13c vector were incubated in an LB medium (+50 µg/mL ampicillin) at 37°C until the OD₆₀₀ value of the cells reached 0.5. The expression of Taar13c was induced by the addition of 1 mM of IPTG, and the cells were incubated for 4 hours.

After incubation, the cells were centrifuged (7000 g, 20 min, 4°C), and pellets obtained through the centrifuge were resuspended in PBS including 2 mM EDTA. Then, the cells were disrupted by sonication (5 s on/off, 5 min) to hemolyze the cells, and centrifuged again (12000 g, 4°C, 20 min).

After repeating the sonication and centrifugation, the pellet of a sample was solubilized in a solubilization buffer (0.1 M Tris-HCl, 20 mM sodium dodecyl sulfate (SDS), 100 mM dithiothreitol (DTT), 1 mM EDTA, pH 8.0). The solubilized protein was dialyzed against a 0.1 M sodium phosphate buffer solution including 10 mM of SDS using a 10 K MWCO dialysis cassette (Thermo Scientific, USA).

Then, filtering was performed using a 0.2 µm bottle top filter (Thermo Scientific, USA), and applied to a HisTrap HP column equilibrated in 0.1 M sodium phosphate (pH 8.0) including 10 mM of SDS. The column was successively washed with a washing buffer (0.1 M sodium phosphate, 10 mM SDS) until pH reached from 8.0 to 7.0. After that, Taar13c was dissolved in the same buffer of pH 6.0 and separated.

The protein thus dissolved and separated was dialyzed against a HEPES buffer II (20 mM HEPES-NaOH, 100 mM NaCl, 25 mM cholate, 1 mM EDTA, pH 8.0). Dialyzed and purified Taar13c was analyzed by SDS-PAGE and western blot analysis.

### <Preparation Example 2>

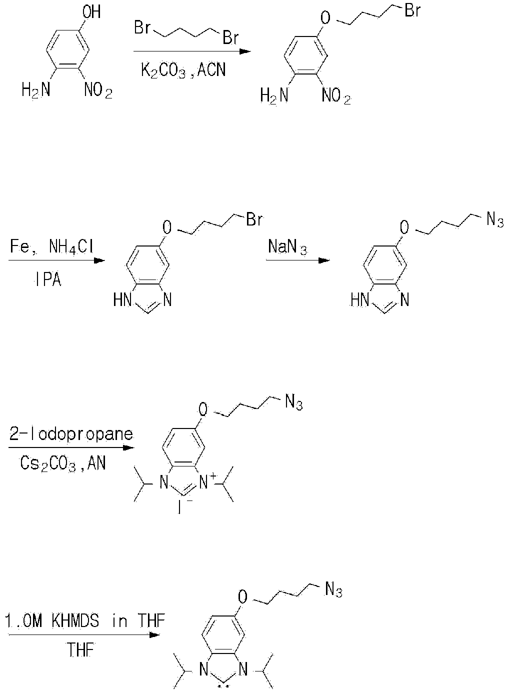

According to the reaction above, Carbene Compound 1 was prepared.

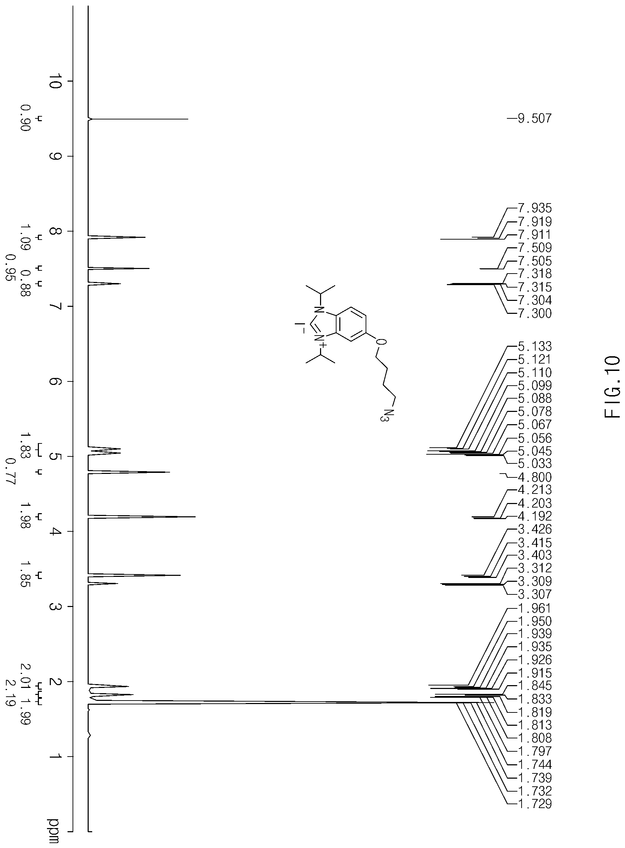

In FIG. 10, ¹H-NMR spectrum of Carbene Compound 1 is shown.

### <Preparation Example 3>

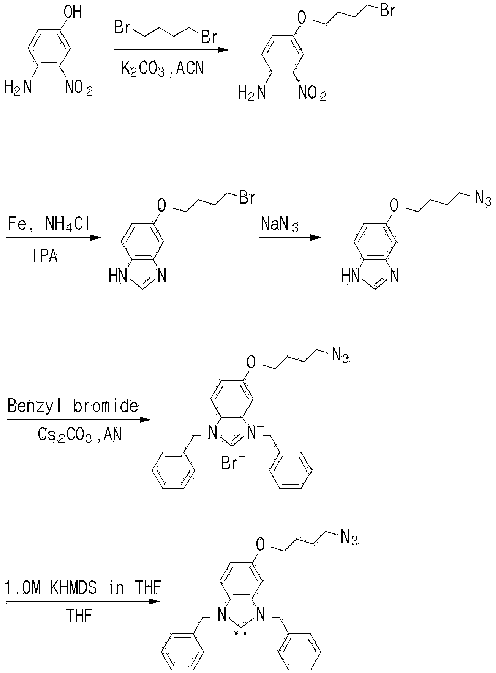

According to the reaction above, Carbene Compound 2 was prepared.

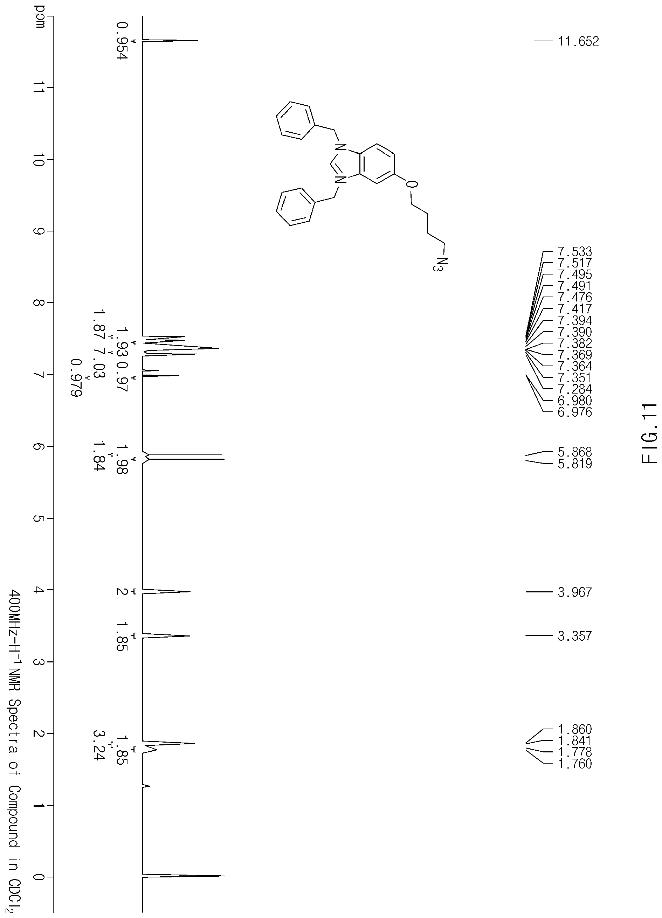

In FIG. 11, ¹H-NMR spectrum of Carbene Compound 2 is shown.

### <Preparation Example 4>

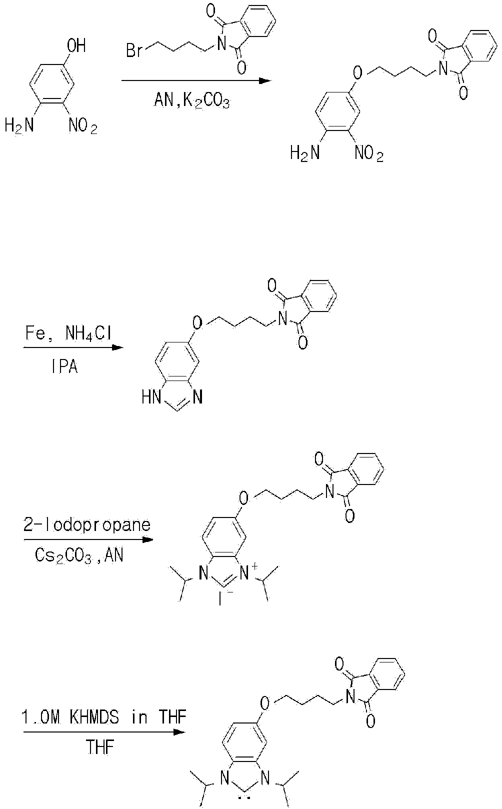

According to the reaction above, Carbene Compound 3 was prepared.

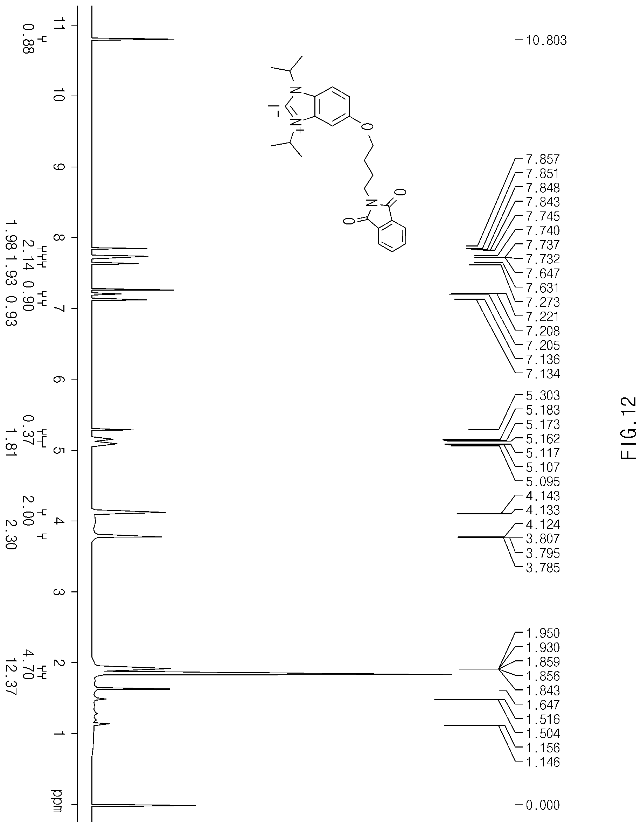

In FIG. 12, ¹H-NMR spectrum of Carbene Compound 3 is shown.

### <Preparation Example 5>

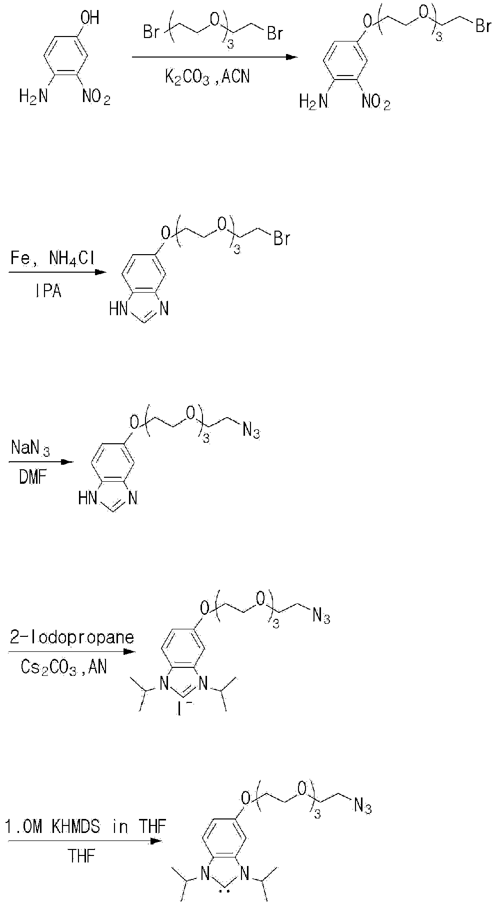

According to the reaction above, Carbene Compound 4 was prepared.

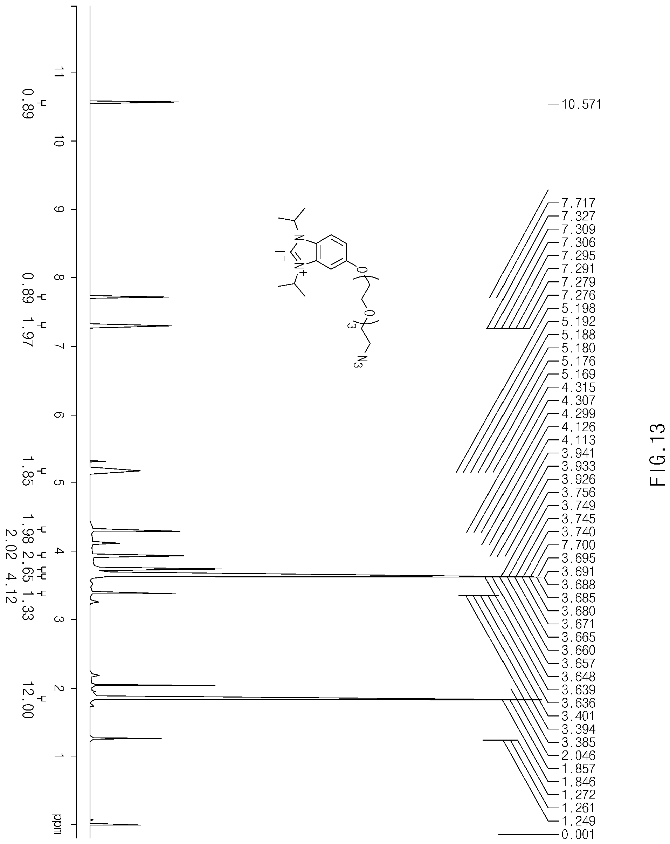

In FIG. 13, ¹H-NMR spectrum of Carbene Compound 4 is shown.

### <Preparation Example 6>

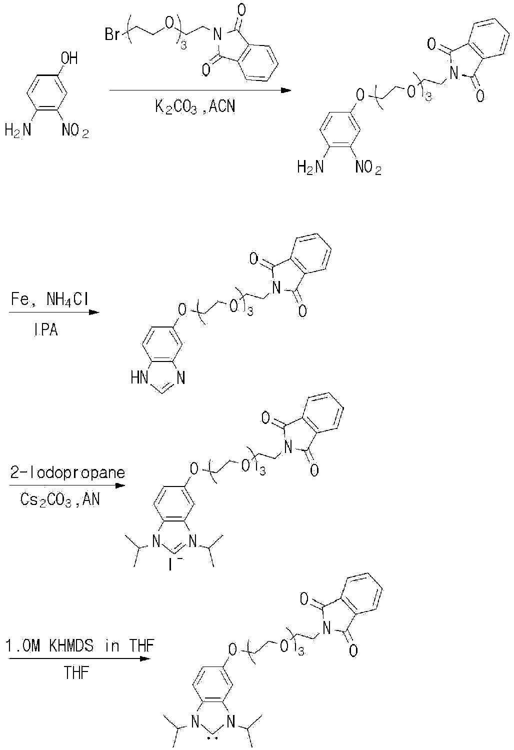

According to the reaction above, Carbene Compound 5 was prepared.

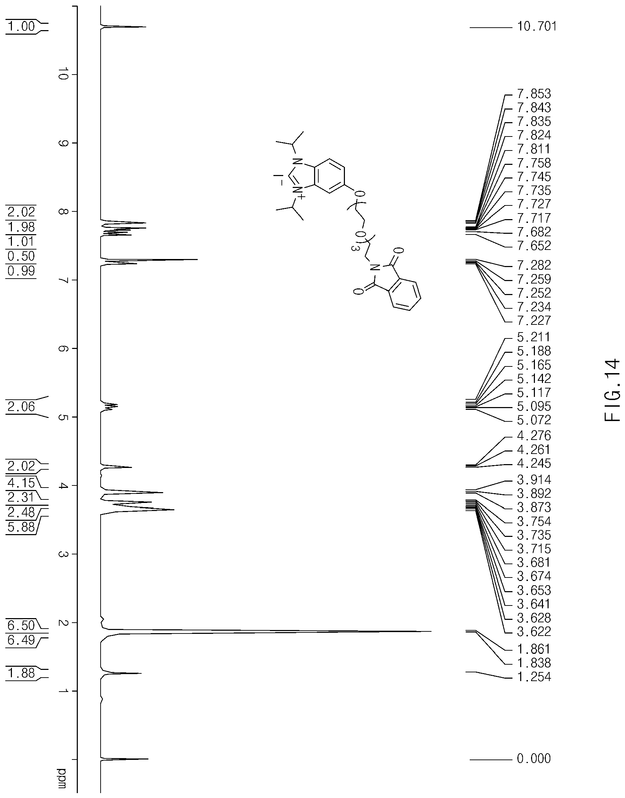

In FIG. 14, ¹H-NMR spectrum of Carbene Compound 5 is shown.

### <Example 1> Manufacture of graphene transistor

### 1-1. Formation of graphene film on substrate

A copper foil was positioned in a chamber, heated to 1,000°C, and maintained in 90 mTorr and 8 sccm of H₂ for 30 minutes (pre-annealing for 20 minutes and stabilizing for 10 minutes). Then, CH₄ was applied in 20 sccm for 40 minutes in a state of a total pressure of 560 mTorr, the temperature was cooled to 200°C in a rate of 35°C/min, and a furnace was cooled to room temperature to form a graphene film (graphene layer) of a single layer on the copper foil.

Then, on the graphene film formed on the copper foil, a polymethyl methacrylate (PMMA, MicroChem Corp, 950 PMMA A4, 4% in anisole) solution was spin coated in a rate of 6,000 rpm per minute, and the graphene film coated with the PMMA was separated from the copper foil using an etchant. The graphene film separated from the copper foil was immersed in de-ionized distilled water for 10 minutes to remove etchant ions remaining on the graphene film.

The graphene film thus washed was transferred to a silicon wafer which was a substrate, and a PMMA solution was dropped on the graphene film to remove PMMA coated on the graphene film to form a graphene film on the substrate. In this case, transparency was maintained to 97.8%.

On the graphene film formed on the substrate, a positive photoresist (AZ5214, Clariant Corp) was spin coated, and the graphene film was patterned through UV exposing, baking and developing processes.

### 1-2. Formation of electrode

At both terminals of the graphene film patterned and aligned as above, a pattern electrode (width/length = W/L = 1, L = 100 µm channel length) was formed through an RIE (oxygen plasma treatment) method, and then, through image reversal, thermal deposition and lift-off processes, a graphene transistor in which an electrode (W/L = 5, L = 100 µm channel length) was formed on a portion of the graphene film, was formed.

### 1-3. Immobilization of cadaverine olfactory receptor

### (Taar13c)

On the graphene film, Taar13c of Preparation Example 1 was added and reacted at 4°C for 12 hours or more to immobilize Taar13c to the graphene film to manufacture a graphene transistor.

### <Example 2> Manufacture of graphene transistor

### 1-1. Formation of graphene film on substrate

A copper foil was positioned in a chamber, heated to 1,000°C, and maintained in 90 mTorr and 8 sccm of H₂ for 30 minutes (pre-annealing for 20 minutes and stabilizing for 10 minutes). Then, CH₄ was applied in 20 sccm for 40 minutes in a state of a total pressure of 560 mTorr, the temperature was cooled to 200°C in a rate of 35°C/min, and a furnace was cooled to room temperature to form a graphene film (graphene layer) of a single layer on the copper foil.

Then, on the graphene film formed on the copper foil, a polymethyl methacrylate (PMMA, MicroChem Corp, 950 PMMA A4, 4% in anisole) solution was spin coated in a rate of 6,000 rpm per minute, and the graphene film coated with the PMMA was separated from the copper foil using an etchant. The graphene film separated from the copper foil was immersed in de-ionized distilled water for 10 minutes to remove etchant ions remaining on the graphene film.

The graphene film thus washed was transferred to a silicon wafer which was a substrate, and a PMMA solution was dropped on the graphene film to remove PMMA coated on the graphene film to form a graphene film on the substrate. In this case, transparency was maintained to 97.8%.

On the graphene film formed on the substrate, a positive photoresist (AZ5214, Clariant Corp) was spin coated, and the graphene film was patterned through UV exposing, baking and developing processes.

### 1-2. Formation of electrode

At both terminals of the graphene film patterned and aligned as above, a pattern electrode (width/length = W/L = 1, L = 100 µm channel length) was formed through an RIE (oxygen plasma treatment) method, and then, through image reversal, thermal deposition and lift-off processes, a graphene transistor in which an electrode (W/L = 5, L = 100 µm channel length) was formed on a portion of the graphene film, was formed.

### 1-3. Formation of linker layer

Carbene Compound 1 (50 mg) of Preparation Example 1 was dissolved in a THF solvent (10 mL) and was added to the graphene film, mixed with 2 mM of fluorinated tetrabutylammonium, and reacted at room temperature for 30 minutes. Then, the graphene film was washed with THF to form a linker layer.

### 1-4. Immobilization of cadaverine olfactory receptor (Taar13c)

The linker layer was treated with 10 µL of a mixture solution of Taar13c of Preparation Example 1 and 3 wt% of DMTMM in a volume ratio of 1:1, reacted at 4°C for 5 hours or more, and washed with a buffer solution to immobilize Taar13c to the linker layer to manufacture a graphene transistor.

### <Comparative Example 1>

A graphene transistor was manufactured by the same method as in Example 2 except for using carbon nanotube instead of the graphene film in Example 2.

### <Experimental Example 1> Measurement results according to cadaverine concentration

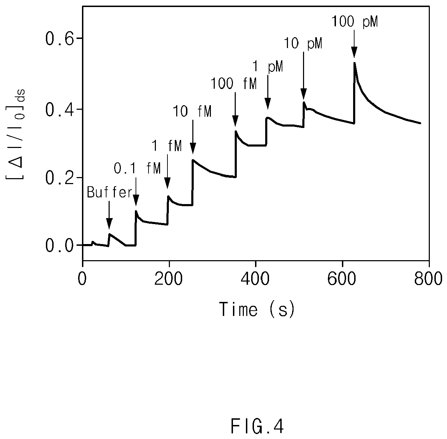

The graphene transistor of Example 2 was prepared, and a detection experiment was conducted by injecting cadaverine in a concentration of 0.1 fM, 1 fM, 10 fM, 100 fM, 1 pM, 10 pM, or 100 pM. The results are shown in FIG. 4.

According to Experimental Example 1 and FIG. 4, it could be confirmed that an electric signal was sensed up to a cadaverine concentration of 0.1 fM by the graphene transistor of the present invention, and a very high sensitivity could be confirmed.

### <Experimental Example 2> Real-time detection results of cadaverine according to the decay of beef according to time

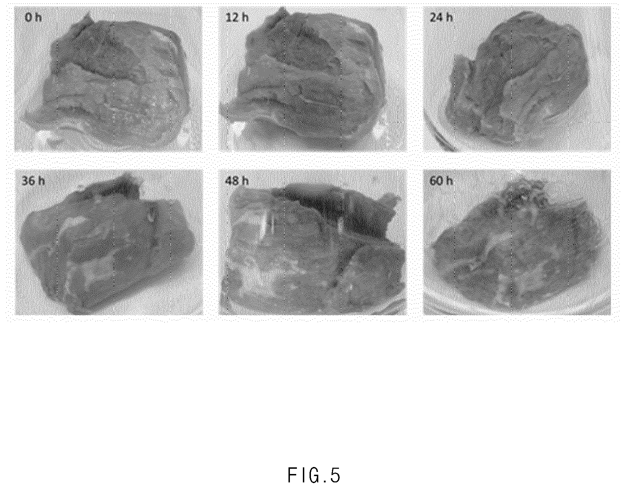

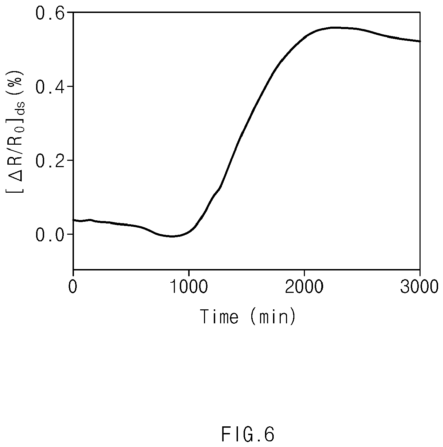

1.8 L of beef was stood at a temperature of 21°C in a humidity of 30%, and observed results from 0 hours to 60 hours are shown in FIG. 5, and detection results using the graphene transistor of Example 2 are shown in FIG. 6.

According to Experimental Example 2 and FIG. 6, it could be confirmed that resistance change was rapid after about 15 hours(900 minutes) by cadaverine generated from the beef.

### <Experimental Example 3> Detection results of graphene transistor for diverse materials

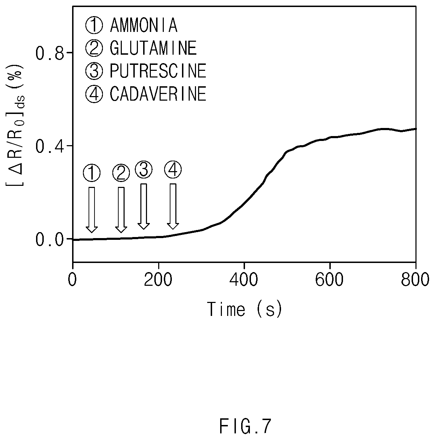

The graphene transistor of Example 2 was prepared, and detection results when the graphene transistor contacted ammonia, glutamine, putrescine, and cadaverine, in order are shown in FIG. 7.

According to Experimental Example 3 and FIG. 7, it could be confirmed that the graphene transistor of the present invention has cadaverine selectivity.

### <Experimental Example 4> Detection results of bio sensor using graphene transistor

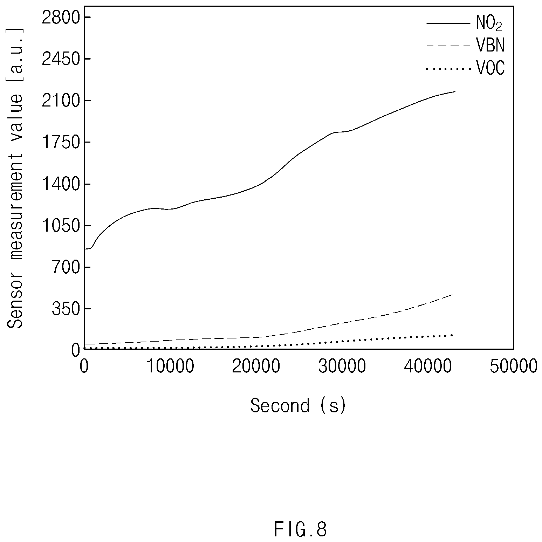

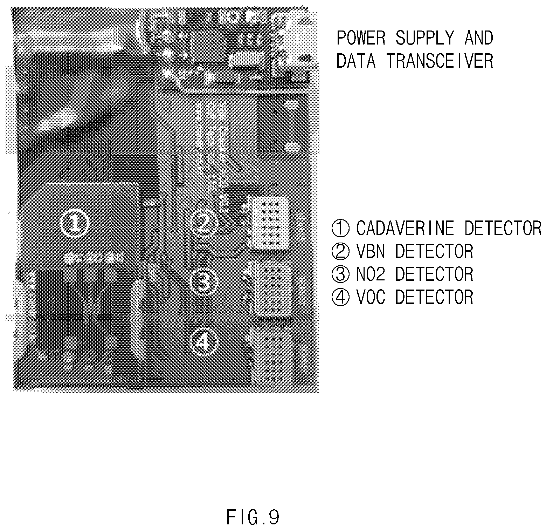

By using the graphene transistor of Example 2 and a bio sensor including commonly used NO₂, VBN, and VOC detectors, manufactured as in FIG. 9, real-time detection results on NO₂, VBN and VOC generated during the decay of the beef of Experimental Example 2 are shown in FIG. 8.

### <Comparative Experimental Example 1>

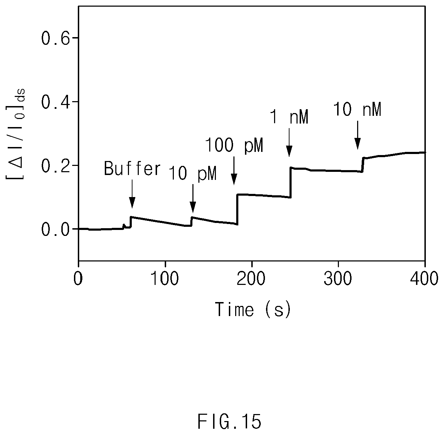

The evaluation results on a cadaverine detection limit using the graphene transistor of Comparative Example 1 are shown in FIG. 15.

According to Comparative Experimental Example 1 and FIG. 15, it could be confirmed that the detection limit was improved by about 100,000 times or more for a case of using the graphene transistor of the present invention when compared to a case of using the graphene transistor of Comparative Example 1.

## Claims

1. A graphene channel member, comprising a graphene film and a cadaverine olfactory receptor immobilized to the graphene film.

2. The graphene channel member according to claim 1, wherein the cadaverine olfactory receptor is immobilized to the graphene film by a physical bond.

3. The graphene channel member according to claim 2, wherein the immobilization by the physical bond is immobilization of the cadaverine olfactory receptor to the graphene film through absorption.

4. The graphene channel member according to claim 1, wherein the cadaverine olfactory receptor is immobilized to the graphene film by a chemical bond.

5. The graphene channel member according to claim 4, wherein the chemical bond is immobilized using a carbene compound represented by the following Formula 1 or Formula 2 as a liker: 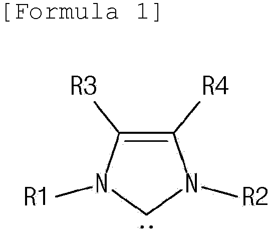 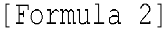 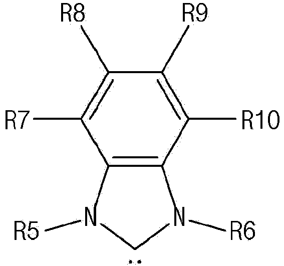 in Formulae 1 and 2,
R1, R2, R5 and R6 are the same or different, and are each independently hydrogen, an alkyl group of 1 to 20 carbon atoms, a cycloalkyl group of 3 to 20 carbon atoms, an aryl group of 6 to 30 carbon atoms, or a heteroaryl group of 2 to 30 carbon atoms,
R3, R4, R7, R8, R9 and R10 are the same or different, and are each independently hydrogen, an alkyl group of 1 to 20 carbon atoms, a cycloalkyl group of 3 to 20 carbon atoms, an aryl group of 6 to 30 carbon atoms, a heteroaryl group of 2 to 30 carbon atoms, or a structure represented by the following Formula 3, or two or more adjacent substituents among R7 to R10 are combined to form a hydrocarbon ring,
at least one of R3 and R4 is a structure represented by the following Formula 3, and
in the case where at least one of R7 to R10 has a structure represented by the following Formula 3, or two or more adjacent substituents among R7 to R10 are combined to form a hydrocarbon ring, at least one hydrogen which is bonded to carbon forming the hydrocarbon ring is substituted with a structure represented by the following Formula 3: 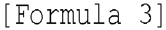 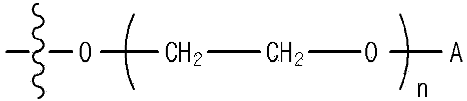
in Formula 3,
n is a repeating number of the unit in a parenthesis and an integer of 1 to 30, and
A is an alkyl group of 1 to 20 carbon atoms, including a nitrogen (N) atom, or a heteroaryl group of 2 to 30 carbon atoms, including a nitrogen (N) atom.

6. The graphene channel member according to claim 1, wherein the cadaverine olfactory receptor reacts with liquid phase cadaverine or gas phase cadaverine.

7. The graphene channel member according to claim 1, wherein the cadaverine olfactory receptor comprises trace amine-associated receptor 13c (Taar13c).

8. The graphene channel member according to claim 1, wherein the graphene film has a single layer or a bi-layer.

9. The graphene channel member according to claim 1, wherein the graphene film is patterned.

10. The graphene channel member according to claim 1, wherein the graphene film has a thickness of 0.1 to 1 nm.

11. A graphene transistor, comprising:
a substrate;
the graphene channel member according to any one of claim 1 to claim 10; and
a pair of electrodes.

12. A bio sensor comprising the graphene transistor of claim 11.
